# EUROPEAN PATENT APPLICATION

(11) **EP 4 473 884 A1**
(43) Date of publication of application: **11.12.2024**
(21) Application number: 23178017.2
(22) Date of filing: 07.06.2023
(51) Int. Cl.: A47J 47/00

(54) **DETECTING PHYSICAL PARAMETERS**

(71) Applicant: Versuni Holding B.V., 5656 AE Eindhoven (NL)
(72) Inventor: MANZONI, Giulio, 5656 AE Eindhoven (NL); VAUPOT, Jan, 5656 AE Eindhoven (NL); HIETBRINK, Ingrid, 5656 AE Eindhoven (NL)
(74) Representative: Vollering, Stefanus Franciscus Maria

(57) **Abstract**

A mechanism for estimating a value of a physical parameter at a sensing location. A pulsed electromagnetic wave is received by a sensing element at the sensing location. The sensing element comprises a capacitive element and resistive element connected in a loop. The impedance of the capacitive element and/or resistive element changes responsive to a change in the physical parameter at/near the sensing element. The loop creates an oscillating electromagnetic wave that changes responsive to the impedance of the capacitive/resistive element(s), and therefore the physical parameter. A detection element monitors the oscillating electromagnetic wave to produce an estimated value of the physical parameter at the sensing location.

## Description

### FIELD OF THE INVENTION

This invention relates to the field of monitoring physical parameters, and in particular, to remote monitoring of physical parameters.

### BACKGROUND OF THE INVENTION

Many cooking and baking devices, such as air fryers, make use of interactive recipes in order to ensure optimized taste and healthiness of the cooked meals. For these devices it is usually important to measure or monitor one or more physical parameters of the food item and/or its surrounding environment. Example parameters include temperature, water content, humidity, pressure, force and so on. As an example, it is usually considered crucial to monitor the temperature of a beef steak during cooking in order to achieve a desired doneness, such as medium, medium-rare, etc.

Even in the absence of interactive recipes, information on such parameters would be useful for an operator of the cooking/baking device. For instance, provision of this information facilitates an operator to control or monitor the cooking progress or completeness.

There is an ongoing desire to improve the accuracy and flexibility of monitoring one or more parameters of the food or its environment.

### SUMMARY OF THE INVENTION

The invention is defined by the claims.

According to examples in accordance with an aspect of the invention, there is provided an apparatus for sensing the value of a physical parameter at a sensing location. The apparatus comprises a transmitter element configured to generate and transmit a pulsed electromagnetic wave; a sensing element, galvanically isolated from the transmitter element and configured to be positioned at or adjacent to the sensing location. The sensing element comprises a capacitive element and a resistive element connected in a loop such that, responsive to the sensing element receiving the pulsed electromagnetic wave, the sensing element generates an oscillating electromagnetic wave that changes responsive to an impedance of the capacitive element and/or resistive element. The capacitive element and/or resistive element is/are configured such that an impedance of the capacitive and/or resistive element changes responsive to changes in the physical parameter in the vicinity of the sensing element. The apparatus further comprises a detection element, galvanically isolated from the sensing element, and configured to detect the oscillating electromagnetic wave and process the detected oscillating electromagnetic wave to estimate a value of the physical parameter at the sensing location.

The present disclosure provides a mechanism for performing remote (short-distance) detection of a physical parameter at a sensing location, e.g., a location of a food item. A pulsed electromagnetic wave is received by a sensing element at the sensing location. The sensing element comprises a capacitive element (i.e., a capacitor) and a resistive element (i.e., a resistor) connected in a loop, e.g., alongside a coil. This creates a resonant signal that oscillates. The oscillations are dependent upon the impedance of the capacitive element and an impedance of the resistive element. The impedance of the capacitive element or resistive element or both is configured to change responsive to changes in the physical parameter (e.g., temperature, humidity, strain, pressure, pH value etc.), so the oscillations will change. The oscillations produce an oscillating electromagnetic wave that can be detected by a detection element. The detected oscillations can then be processed to derive or determine a value for the physical parameter.

The proposed technique thereby provides a mechanism for performing remote detection of a physical parameter at a sensing location within needing to directly power (e.g., using wired power) a device at the sensing location. This increases a flexibility in placement or usage of the detection element.

The detection element may be configured to calculate the time damping constant of the detected oscillating electromagnetic wave and process the calculated time damping constant to estimate the value of the parameter at the sensing location. Different values of the time damping constant relate to different values of the parameter at the sensing location. The time damping constant is independent of the signal strength of the oscillating electromagnetic wave, such that accurate determination of the value can be achieved at arbitrary distances.

The detection element may be configured to integrate a reference signal between two time points defined by an upper or lower envelope of the detected oscillating electromagnetic wave to produce an integrated value for the oscillating electromagnetic wave and process the integrated value to estimate the value of the parameter at the sensing location.

In particular, the time points at which the upper or lower envelope cross two (different) predetermined thresholds may be identified. The integrated value may be produced by integrating a reference signal between these two time points.

In some examples, the transmitter element comprises a transmitter coil for transmitting the pulsed electromagnetic wave; and the detection element comprises a detector coil for detecting the oscillating electromagnetic wave.

In some examples, the transmitter coil and detection coil are together formed from a single coil for transmitting the pulsed electromagnetic wave and detecting the oscillating electromagnetic wave. This advantageously reduces the number of components needed to form the apparatus. This embodiment appreciates that after the pulsed electromagnetic wave is produced, the transmitter coil will remain idle during the oscillations of the sensing circuit. Accordingly, it has been identified that the transmitter coil can be repurposed for use as the detection coil in this time.

The resistive element may be a thermistor, such that the physical parameter is a temperature. This advantageously provides a temperature detection system.

In some examples, the pulsed electromagnetic wave is a square pulsed electromagnetic wave. A square pulsed electromagnetic wave provides a reliable, repeatable and consistent burst of energy for triggering the oscillations of the sensing element.

The sensing element may comprise a sensing coil connected in the loop with the capacitive element and the resistive element for receiving the pulsed electromagnetic wave and outputting the oscillating electromagnetic wave. Use of a sensing coil increases the sensitivity of the sensing element to the pulsed electromagnetic wave.

There is also proposed a cooking device comprising any herein disclosed apparatus, in which the sensing element is positioned such that the sensing location is in or in the vicinity of a food item in the cooking device and/or a food item being cooked (or to be cooked) by the cooking device.

The cooking device may comprise a pan for housing the food item.

The transmitter element and the detection element may be positioned externally to the pan and the sensing element may be configured to be housed within the pan.

Preferably, the pan is non-metallic. Use of a non-metallic pan reduces any attenuation of the pulsed or oscillating electromagnetic wave, thereby improving signal strength. By way of example, the pan may be made from ceramic, plastic and/or glass.

The cooking device may further comprise a housing and a moveable support for supporting the pan, wherein the housing and moveable support are configured such that the moveable support, when supporting the pan, is able to move between: a first position in which any food item housed in the pan, is enclosed by the housing, the pan and the moveable support; and a second position in which the pan is exposed for accessing any food item housed in the pan.

In some examples, where present, the transmitter coil and the detector coil are mounted to the moveable support. In some examples, the transmitter coil and the detector coil are integrated into or positioned on the housing.

In at least one embodiment, the pan comprises a planar structure configured, in use, to support the food item in the cooking device, the planar structure lying in a first plane; and the transmitter coil and detector coil are positioned to lie in a second plane that is perpendicular to the first plane.

These and other aspects will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
FIG. 1 illustrates a proposed apparatus;
FIG. 2 illustrates another proposed apparatus;
FIG. 3 illustrates a pulsed electrical signal or pulsed electromagnetic wave;
FIG. 4 illustrates a sensing electrical signal or oscillating electromagnetic wave;
FIG. 5 illustrates an integration of an envelope of a detection electrical signal;
FIG. 6 illustrates a proposed cooking device;
FIG. 7 illustrates a portion of another proposed cooking device;
FIG. 8 illustrates a first configuration for transmitter and detector coils for a proposed cooking device;
FIG. 9 illustrates a second configuration for transmitter and detector coils for a proposed cooking device;
FIG. 10 illustrates a configuration for a transmitter-detector coil for a proposed cooking device;
FIG. 11 illustrates another configuration for the transmitter and detector coil(s) for a proposed cooking device; and
FIG. 12 illustrates yet another configuration for the transmitter and detector coil(s) for a proposed cooking device.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

Proposed approaches provide a mechanism for estimating a value of a physical parameter at a sensing location. A pulsed electromagnetic wave is received by a sensing element at the sensing location. The sensing element comprises a capacitive element and resistive element connected in a loop. The impedance of the capacitive element and/or resistive element changes responsive to a change in the physical parameter at/near the sensing element. The loop creates an oscillating electromagnetic wave that changes responsive to the impedance of the capacitive/resistive element(s), and therefore the physical parameter. A detection element monitors the oscillating electromagnetic wave to produce an estimated value of the physical parameter at the sensing location.

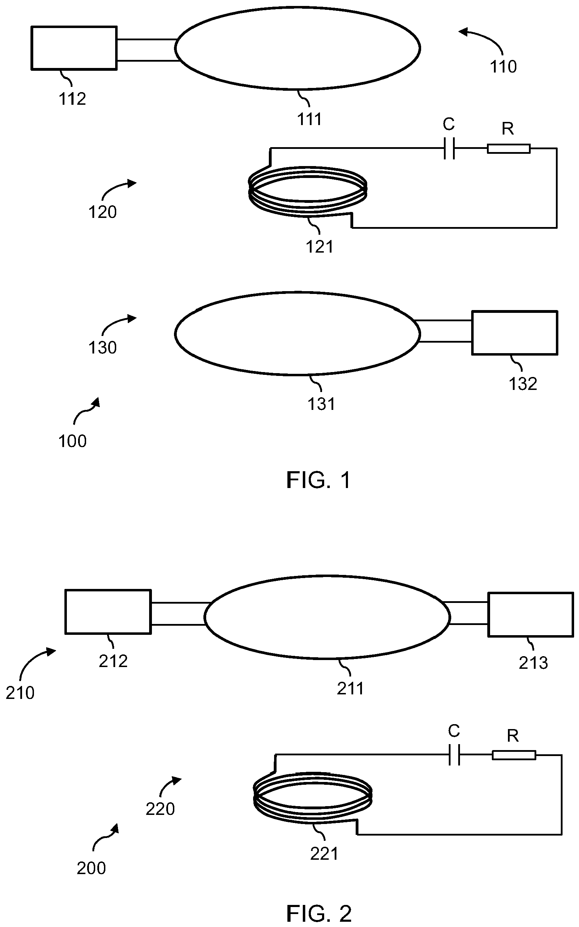

FIG. 1 illustrates an apparatus 100 according to a proposed approach. The apparatus is designed for sensing the value of a physical parameter at a sensing location.

The apparatus comprises a transmitter element 110, a sensing element 120 and a detection element 130. In this example, the transmitter element 110 and detection element 130 are formed as separate, distinct components.

The transmitter element 110 is configured to generate and transmit a pulsed electromagnetic wave. More particularly, the transmitter element 110 comprises a transmitter coil 111 that acts as an antenna for outputting the pulsed electromagnetic wave. Any other form of antenna could also be used instead of the transmitter coil 111. The transmitter element also comprises a signal generator 112 that generates a pulsed (electrical) signal that is received by the transmitter coil 111. The transmitter coil receives the pulsed (electrical) signal and generates the pulsed electromagnetic wave.

The sensing element 120 is galvanically isolated from the transmitter element. The sensing element 120 is positioned at or adjacent to the sensing location. The purpose of the sensing element is to produce an oscillating electromagnetic wave responsive to the pulsed electromagnetic wave generated by the transmitter element. More particularly, one or more properties of the oscillating electromagnetic wave change responsively to changes in the physical parameter at the sensing location. By monitoring the oscillating electromagnetic wave, it is therefore possible to determine a value of the physical parameter.

To achieve this purpose, the sensing element 120 comprises a capacitive element C (e.g., a capacitor) and a resistive element R connected in a loop. A pulsed electromagnetic wave received by the sensing element will thereby oscillate or resonate through the loop formed in the sensing element, producing an oscillating electromagnetic wave. One or more properties of the oscillating electromagnetic wave will change responsive to any changes in impedance of the capacitive element and/or resistive element. The capacitive and/or resistive element(s) is/are configured to change impedance responsive to a change in the physical parameter. In this way, one or more properties of the oscillating electromagnetic wave will change responsive to a change in the physical parameter.

Preferably, (only) the resistive element is configured to change impedance (specifically, resistance) responsive to the change in the physical parameter. A resistance of a resistive element will typically change proportionally to a change in the physical parameter when designed to do so, increasing an ease of determining the value of the physical parameter.

To improve the sensitivity of the sensing element to the pulsed electromagnetic wave and to increase the strength of the oscillating electromagnetic wave, the sensing element 120 may comprise a sensing coil 121 connected in the loop with the capacitive element C and the resistive element R. The sensing coil 121 acts as an antenna for receiving the pulsed electromagnetic wave and producing the oscillating electromagnetic wave. Any other form of antenna could also be used instead of the sensing coil 121.

The detection element 130 is galvanically isolated from the sensing element 120 and is configured to detect the oscillating electromagnetic wave. This can be achieved using a detector coil 131 for detecting the oscillating electromagnetic wave. The detector coil acts as an antenna to produce an electrical signal responsive to the oscillating electromagnetic wave. Any other form of antenna could also be used instead of the detector coil 131.

The detection element 130 is configured to process the detected oscillating electromagnetic wave to estimate a value of the physical parameter at the sensing location. To do so, the detection element may comprise processing circuitry 132 configured to process the received oscillating electromagnetic wave (e.g., the electrical signal produced responsive to the oscillating electromagnetic wave) in order to estimate a value of the physical parameter. More particularly, the processing circuitry 132 is configured to determine a value for one or more properties of the oscillating electromagnetic wave (that is/are changed responsive to a change in the physical parameter at the sensing location) and estimate the value of the physical parameter from such properties. Suitable examples of these procedures are given later in this disclosure.

More particularly, the detection element may comprise a detector coil 131 that produces a detection electrical signal responsive to any electromagnetic wave received at the detector coil. This produces a detection electrical signal responsive to the oscillating electromagnetic wave. In particular, the amplitude (e.g., voltage) of the detection electrical signal may vary proportionally to the amplitude of the oscillating electromagnetic wave.

The processing circuitry 132 of the detection element 130 may process the detection electrical signal using analogue and/or digital circuitry in order to estimate the value of the physical parameter.

As previously explained, the capacitive element C and/or resistive element R of the sensing element 120 is/are configured to change impedance responsive to changes in the physical property at the sensing location. A number of suitable examples of appropriate capacitive/resistive elements that change impedance in this way are hereafter described.

In one example, the resistive element is a thermistor (also known as a resistance thermometer). The resistance of a thermistor will change responsive to a change in temperature at the sensing location, such that the oscillating electromagnetic wave will change responsive to said change in temperature. Thus, the sensed physical parameter may be a temperature.

As another example, the capacitive element may be capacitance temperature sensor, whose capacitance changes responsive to a change in temperature. One example is provided by US Patent No. US 4,883,366, although others are known in the art. In this way, the sensed physical parameter may be a temperature.

In another example, the resistive element is a humistor, being a resistor whose resistance varies responsive to a humidity. Suitable examples of humistors are provided by US Patents No. US 4,086,556; US 4,464,647 or US 4,751,022. The resistance of a humistor will change responsive to a change in humidity at the sensing location, such that the oscillating electromagnetic wave will change responsive to said change in humidity. Thus, the sensed physical parameter may be a humidity.

In another example, the capacitive element is a capacitive humidity sensor. Examples of such sensors are provided by US Patents No. US 5,283,711; US 4,651,121 or US 4,164,868. The capacitance of such a capacitive humidity sensor will vary responsive to a humidity at the sensing location, such that the oscillating electromagnetic wave will change responsive to a change in humidity. Thus, the sensed physical parameter may be a humidity.

In another example, the resistive element is a strain gauge, which changes resistance responsive to a strain applied to the stain gauge. Thus, the sensed physical parameter may be a measure of strain at the sensing location. This can, for instance, be useful for determining a measure of weight (e.g., of an item positioned) at the sensing location.

In yet another example, the resistive element may simply be responsive to a resistance at the sensing location, e.g., comprise two electrodes to be positioned - a resistance between which changes responsive to a resistance at the resistance location. This can, for instance, be useful for determining or estimating a measure of a water content (e.g., of an item positioned) at the sensing location and/or pH value at the sensing location.

The examples of potential capacitive or resistive elements that may be used is non-exhaustive, and the skilled person would readily appreciate that other forms of capacitive and/or resistive element could be used dependent upon the desired physical parameter to detect.

The value produced by the detection element may require further calibrating, mapping or processing to accurately estimate a value of the physical parameter. This can be performed by mapping the value produced by the detection element, e.g., using a look-up table or the like, to a true value. Other procedures may make use of one or more mapping functions.

The sensing element is thereby galvanically isolated from the transmitter element and the detection element. This facilitates flexible and wireless positioning of the sensing element away from the other elements of the apparatus 100. The avoidance of wires for directly powering the sensing element advantageously reduces or avoids wires needing to connect to the sensing location, e.g., which may otherwise fray or be damaged during cooking or the like. Thus, more flexible positioning and/or usage of the sensing element is achieved.

FIG. 2 illustrates an alternative apparatus 200, which is still designed for sensing the value of a physical parameter at a sensing location.

The apparatus 200 differs from the previously described apparatus in that the transmitter element and the detector element are combined into a dual transmitter-detector element 210.

Thus, the dual transmitter-detector element comprises a signal generator 212 for producing the electrical signal that generates the pulsed electromagnetic wave and processing circuitry 213 that processes an electrical signal produced responsive to the oscillating electromagnetic wave to determine or derive a value of the physical parameter.

Of course, the dual transmitter-detector element 210 may comprise a single coil 211 for transmitting the pulsed electromagnetic wave and receiving the oscillating electromagnetic wave. The single coil effectively represents the combination of the transmitter coil and the detector coil of the previously described embodiment. The coil 211 may be replaced by any other form of antenna.

The sensing element 220 may be embodied as previously described, e.g., comprising a capacitive element C and a resistive element R connected in a loop - preferably together with a sensing coil 221.

In a preferred embodiment, the single coil 211 of the transmitter-detector element and the sensing coil 221 are positioned to be concentric with one another. This improves the signal-to-noise ratio.

In preferred examples of any herein described apparatus, all coils of any element are positioned to lie parallel to one another, i.e., to have the same orientation. This improves the signal-to-noise ratio.

Any herein described coil or antenna may be formed from any suitable material having suitable electromagnetic characteristics, such as copper or aluminum.

Although illustrated in the form of ellipse or circle, each coil or antenna may be formed in a different shape, e.g., to have a square or oblong cross-sectional shape.

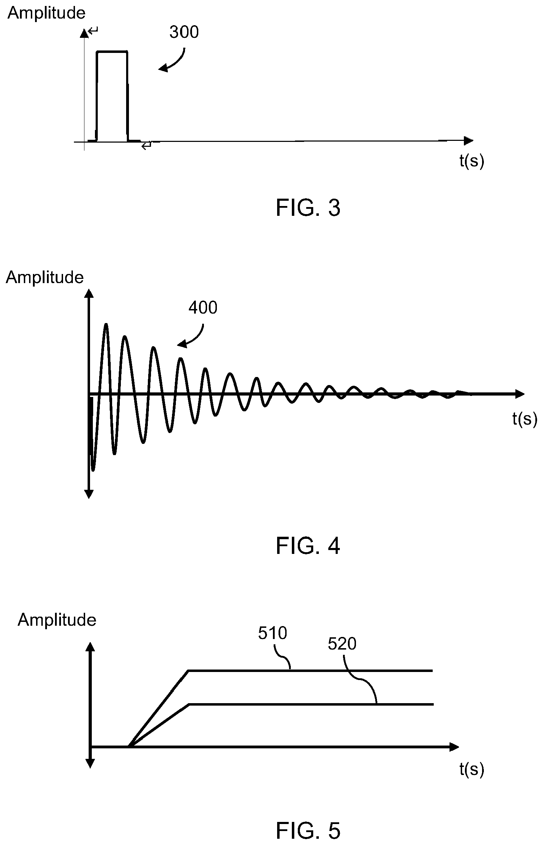

More detail on approaches for estimating a value of the physical parameter, using the proposed technique, at the sensing location are hereafter described with reference to FIGS. 3 to 5, which illustrate various waveforms.

FIG. 3 is a waveform illustrating an example pulsed electromagnetic wave 300 produced by the transmitter element.

In this example, the pulsed electromagnetic wave is a single square-wave pulse. This can be produced by a signal generator producing a pulsed electrical signal which is then output (e.g., by an antenna/coil) in the form of a pulsed electromagnetic wave.

The waveform illustrated by FIG. 3 can therefore represent both the waveform of the pulsed electrical signal (that produces the pulsed electromagnetic wave) or the pulsed electromagnetic wave itself.

FIG. 4 is a waveform illustrating an example oscillating electromagnetic wave 400 produced by the sensing element responsive to the pulsed electromagnetic wave.

As previously explained, the sensing element comprises a capacitive element and resistive element (optionally with a sensing coil) connected in a loop. This effectively forms an oscillating circuit. When the pulsed electromagnetic wave is received by the sensing element, an electrical signal (a "sensing electrical signal" or "oscillating electrical signal") cyclically runs through the loop formed in the sensing circle probe circuit. Each cycle or pass of the loop has less energy, e.g., decays exponentially. The sensing element thereby produces an oscillating electromagnetic wave that decays over time.

The waveform illustrated by FIG. 4 can therefore represent both the waveform of the sensing electrical signal in the loop of the sensing element (that produces the oscillating electromagnetic wave) or the oscillating electromagnetic wave itself.

The speed of the decay or decay time of the sensing electrical signal and/or oscillating electromagnetic wave will change, e.g., proportionally, responsive to the impedance of the capacitive element and/or the resistive element. In particular, the rate of amplitude decay (over time) will change proportionally to the impedance of the capacitive element and/or resistive element. For instance, a Q factor or quality factor or "half-life" defined from the oscillating electromagnetic wave will change, e.g., proportionally, responsive to the impedance of the capacitive element and/or resistive element (and therefore any change in the physical parameter to be monitored).

The detection element is configured to receive this oscillating electromagnetic wave, e.g., producing a detection electrical signal responsive thereto. The detection electrical signal is then processed to determine a measure or value for the physical parameter. The waveform illustrated by FIG. 4 can therefore similarly represent a waveform of the detection electrical signal produced by the detection element before further processing by the detection element to estimate the value of the physical parameter.

In particular a value of the change in voltage of the detection electrical signal over a change in time is indicative of (e.g., proportional to) the measure or value for the physical parameter.

As a working example, (a portion of) an upper or lower envelope of the detection electrical signal may be used to define an integration time domain by the detection element (e.g., by integrating electronics). Integrating a reference signal within this time domain produces a signal that changes proportional to the measure or value of the physical parameter. The portion of the upper or lower envelope may be a portion between two predetermined reference values for the detection electrical signal.

As another working example, a Q factor of the oscillating electromagnetic wave may be determined from the detection electrical signal. The Q factor will change proportional to the measure or value of the physical parameter.

FIG. 5 illustrates a first 510 and second 520 waveform produced by integrating a reference signal during a time interval defined by an upper (or lower) envelope of different detection electrical signals produced from different oscillating electromagnetic waves. The value of the integrated signal on the time interval defined by the envelope of oscillations is responsive to the decay time of the oscillating electromagnetic wave, and therefore the impedance of the capacitive/resistive element(s) and therefore any changes in the physical parameter to be monitored.

More particularly, each waveform may be produced by determining a value that represents the exponential decay of the upper/lower envelope of the respective detection electrical signal. This can be measured by determining the time interval between two crossings of the detection electrical signal at different reference amplitudes. To avoid using timers a voltage proportional to the time interval can be produced. This can be achieved by integrating a given reference signal along the time interval defined by the detection electrical signal between two predetermined reference amplitudes or values. The predetermined reference values may be related by a known ratio.

Thus, each waveform may be determined by a time integration on an interval limited by an upper or lower envelope of a respective detection electrical signal between two predetermined amplitude levels of the upper or lower envelope.

Advantageously, the value of the integrated time interval corresponding to the envelope of oscillations (or determined Q factor) between two reference levels will be independent of the amplitude of the oscillating electromagnetic wave. This means that the proposed technique is able to determine or detect a value for the physical parameter even if signal strength is low and/or for arbitrary distances between the elements.

The first 510 and second 520 waveforms are produced by processing detection electrical signals produced using a sensing element (with a thermistor) positioned in different temperature environments. The environment used to produce the first waveform 510 has a higher temperature than the environment used to produce the second waveform 520.

It will, however, be appreciated that the relationship between characteristics of the waveform and temperature (or other measured parameter) will depend upon the nature of the capacitive and/or resistive element. For instance, some thermistors may increase in impedance with increased temperature, whereas others will reduce impedance. This will have a corresponding impact on the Q factor of the oscillating electromagnetic wave.

The processing of the detection electrical signal may be performed using analogue processing and/or digital processing.

In a working example, the detection element may comprise analogue and/or digital circuitry configured to identify an upper envelope of a received oscillating electromagnetic wave; compare the upper envelope with reference values to identify crossings of the reference values; calculate the time interval between the crossings of reference values; express the time interval as a voltage signal and provide a stable output voltage for further processing. The reference values may be related by a known ratio. This approach produces an output voltage that changes responsive to the Q factor of the oscillating electromagnetic wave, and thereby represents a measure of the physical parameter to be monitored.

The output voltage may, for instance, be mapped or correlated to a value of or for the physical parameter, e.g., which can be predetermined using known quantities or through calibration techniques.

However, other suitable techniques and processes for extracting a measure of a change in amplitude (of the oscillating electromagnetic wave) over a change in time will be apparent to the skilled person. The above-described technique merely provides one working example of such a process.

Any proposed apparatus may be advantageously used in or with a food processing device, such as a cooking device. Examples of cooking devices include an airfryer, pressure cooker, rice cooker, halogen oven, oven, roaster, deep fryer, steamer, hob and so on. Examples of other food processing devices include a food processor, blender, slicer, chopper, grinder and so on.

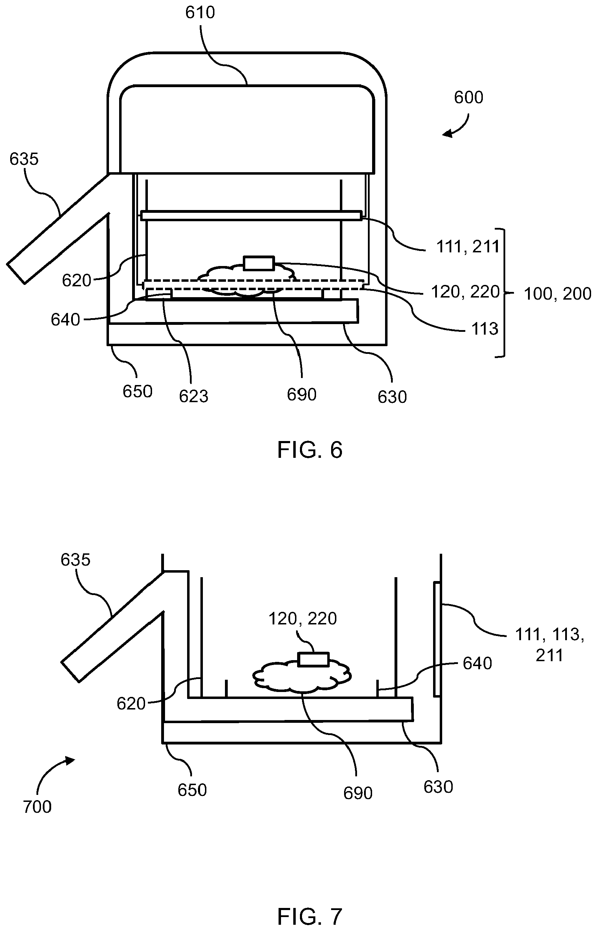

FIG. 6 illustrates an exemplary food processing device 600, namely a cooking device 600 in the form of an airfyer, that comprises a herein described apparatus 100, 200. The sensing element 120, 220 is positioned such that the sensing location is in or in the vicinity of a food item 690 in the cooking device and/or to be cooked (or being cooked) by the cooking device.

The cooking device 600 may comprise cooking/processing circuitry 610 for cooking any food item in the cooking device. Cooking may comprise controlling a heat provided to the food item, a moisture/water/humidity level provided to the food item, a pressure applied to the food item and so on. A wide variety of different functions can be used to control the cooking of food, as would be readily apparent to the skilled person. Preferably, cooking includes at least heating or processing the food item.

The cooking/processing circuitry 610 may be communicatively coupled to the apparatus 100, 200 to receive the estimated value of the physical parameter, which here represents a parameter of or in the vicinity of the food item. The cooking/processing circuitry 610 may control the cooking of the food item responsive to the estimated value. This advantageously provides feedback control over the properties of the food item.

For instance, if the apparatus 100, 200 monitors a temperature, then the cooking/processing circuitry may be able to monitor the temperature using the estimated value in order to control heating of the food item to or at a particular temperature.

As another example, if the apparatus 100, 200 monitors a pressure in the vicinity of the food item, then the cooking/processing circuitry may be able to monitor the pressure using the estimated value in order to control or maintain the pressure applied to the food item to/at a particular pressure.

Of course, the precise control performed by the cooking/processing circuitry 610 (if performed at all) will depend upon the capabilities of the cooking/processing circuitry, and therefore the nature/type of the cooking device.

Approaches for controlling the cooking (e.g., heating) of food responsive to a monitored physical parameter of the food or the food environment are well established in the art. Non-exhaustive examples include the approaches disclosed by European Patents No. EP 3,403,468; EP 3,152,633; EP 3,209,174; EP 3,622,222; EP 3,319,490 or EP 3,993,676; European Patent Application having publication number EP 3 939 480 A1 or US Patent No. 6,753,027. A wide variety of other techniques or procedures will be readily apparent to the suitably skilled person.

However, it is not essential that the cooking/processing circuitry control the cooking of the food item responsive to the predicted/estimated value of the physical parameter. Rather, this estimated value may be presented to an operator of the cooking device (e.g., at a user interface) to facilitate manual control over the cooking device, e.g., manual control over termination of the cooking process, temperature control, humidity control and so on.

It will be appreciated that automated (feedback) control over cooking of the food item using the estimated value of the physical parameter and provision of a visual representation of the estimated value of the physical parameter are not mutually exclusive. Thus, in some examples, the cooking device may be configured to control cooking of the food item using the estimated value of the physical parameter and provide of a visual representation of the estimated value of the physical parameter, e.g., at a display.

It will therefore be appreciated that the cooking device 600 may comprise a display (not shown) for providing a visual representation of the estimated value of the physical parameter.

As previously mentioned, it may be necessary to map or further process the value produced by the apparatus 100, 200 to produce an accurate estimation of the physical parameter. This can be performed by the cooking/processing circuitry 610.

The cooking device may comprise a pan 620 for housing the food item in the cooking device. In some examples, the pan 620 supports a basket 640 for supporting the food.

In preferred examples, and as illustrated, the transmitter element 111, 211 and the detection element 113, 211 of the apparatus 100, 200 are positioned externally to the pan 620. Of course, the sensing element 120, 220 is configured to be housed within the pan such that the sensing location is a location of the food item.

The cooking device 600 may further comprise a moveable support 630 for the pan. The moveable support supports the pan and is able to move the position of the pan, e.g., to insert the pan 620 into the cooking device or expose the pan.

More particularly, the cooking device may comprise a housing 650. The housing 650 and the moveable support 630 may be configured such that the moveable support, when supporting the pan 620, is able to move between: a first position in which any food item housed in the pan, is enclosed by the housing, the pan and the moveable support; and a second position in which the pan is exposed for accessing any food item housed in the pan. The movement between the first and second positions may be controlled or controllable by an individual.

This approach effectively allows for accessing of the interior of the pan for placing the food item (e.g., before cooking) or removal of the food item (e.g., after cooking).

The moveable support 630 may comprise a handle 635 for ease of movement by an individual.

Preferably, the pan 630 is non-metallic, e.g., made of plastic. This reduces the attenuation of any electromagnetic waves transmitted through the pan, to thereby increase the signal strength of the electromagnetic waves.

If present, preferably the basket 640 is (e.g., also) non-metallic, e.g., made of plastic. This reduces the attenuation of any electromagnetic waves transmitted to or from the sensing element, to thereby increase the signal strength of the electromagnetic waves.

In the illustrated example, the pan 620 comprises a planar structure 623 configured to, in use, support the food item in the cooking device. The planar structure 623 is configured to, when the cooking device is in use, lie generally parallel with a horizontal plane (e.g., perpendicular to a direction of gravity). It is not, however, essential that the planar structure actually supports the food item in the cooking device. The planar structure 623 defines a first plane.

In the illustrated example, the transmitter and detector coil(s) 111, 113, 211 of the apparatus 100, 200 lie parallel to this first plane. Thus, the transmitter and detector coils are located in a generally horizonal plane.

The coil(s) may be directly wound around the pan 620.

Alternatively and/or additionally, the coil(s) may be mounted to the moveable support, such that they move with the moveable support. In such embodiments, it may be necessary to have a particular cable connection between the coil(s) and the other element(s) of the apparatus and/or cooking/processing circuitry that is able to adapt to a change in position of the moveable support without becoming disconnected. This cable connection may be configured to have a spiral and/or harmonica shape. Alternatively, the cable connection may be in the form of a cable drum that automatically extends and/or retracts a length of cable/wire with a movement of the moveable support.

FIG. 7 illustrates a portion of a cooking device 700 having an alternative arrangement for the transmitter and detector coil(s) 111, 113, 211 of the apparatus 100, 200. The cooking device 700 is otherwise identical to the cooking device 600 described.

In particular, the transmitter and detector coil(s) 111, 113, 211 may be positioned to lie in a second plane that is perpendicular to the first plane, i.e., to lie vertically. The coil(s) may be coupled to or positioned on the housing 650 and/or integrated therein. This is facilitated by the vertical orientation of the coil(s).

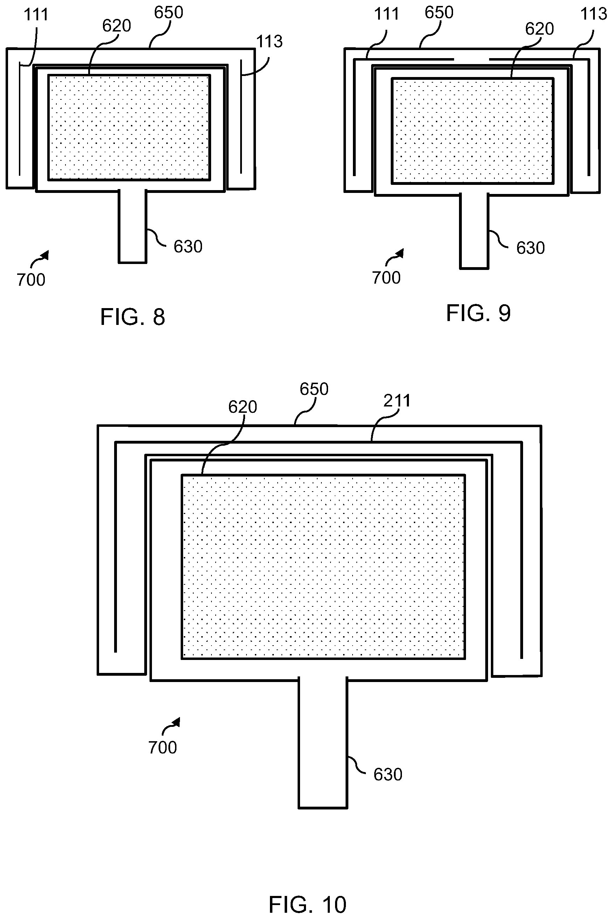

FIGS. 8, 9 and 10 illustrates various configurations and arrangements for the transmitter and detector coil(s) 111, 113, 211 according to different embodiments. Each of FIGS. 8, 9 and 10 provide a cross-sectional top-down view of the cooking device 700.

FIG. 8 illustrates a configuration in which the transmitter 111 and detector 113 coils are separate and distinct from one another, and are positioned to occupy a single side of the housing. For instance, the coils may be respectively positioned on (part of) a left wall and (part of) a right wall of the housing 650. The two coils lie in a respective plane, and are positioned to lie parallel to one another.

Each coil may be replaced by another form of antenna.

FIG. 9 illustrates an alternative configuration in which the transmitter 111 and detector 113 coils are separate and distinct from one another but are each positioned to extend around more than one side of the housing. For instance, the transmitter coil may be positioned on (part of) a left and (part of) a back wall of the housing and the detector coil may be positioned on (part of) a right (and part of) the back wall of the housing. This increases the range of the coil(s) for improved reliability. Each coil may be replaced by another form of antenna.

FIG. 10 illustrates another alternative configuration in which the transmitter and detector coils are formed from a single coil 211. The single coil 211 surrounds three sides of the pan 620. The coil may be replaced by another form of antenna.

In all the embodiments illustrated by FIGS 8, 9 and 10, the coil(s) are integrated into the housing. In other examples, the coils may be positioned on the housing, e.g., between the housing 650 and the pan 620.

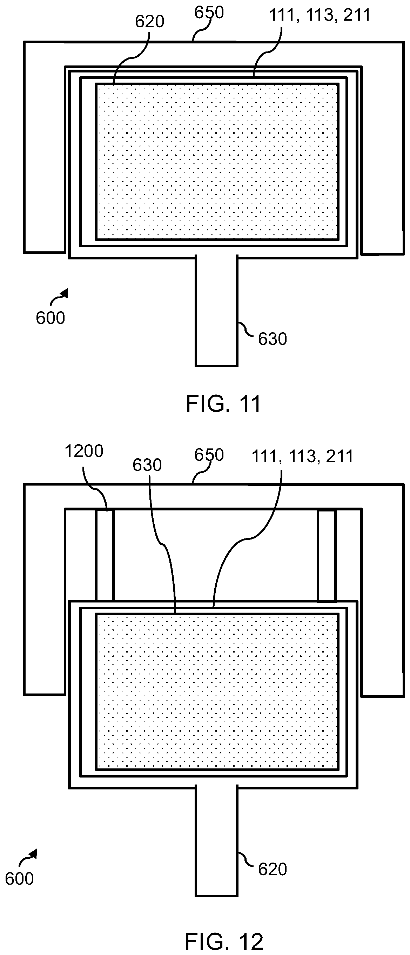

FIGS. 10 and 11 illustrates a variant in which the transmitter and detector coil(s) 111, 113, 211 are positioned around the pan 620. In particular, the coil(s) are positioned on the movable support 630 to surround the pan, which is also illustrated in FIG. 6.

The moveable support 630 is configured to move along one or more rails 1200 so as to move the pan between a first position (illustrated in FIG. 10) and a second position (illustrated in FIG. 11). In the first position, the housing 650, the pan 620 and the moveable support 630 enclose any food item housed in the pan. In the second position, any food item housed in the pan is housed, e.g., for accessing by an operator of the cooking device 600.

As previously explained, the transmitter and detector coil(s) 111, 113, 211 may be formed from separate, dedicated coils. Alternatively, the transmitter and detector coil(s) may be formed from a single coil.

The skilled person would appreciate how the proposed apparatus can be adapted for use with other forms of food processing device. In general, the proposed apparatus can be used to determine a measure of a parameter in the vicinity of food to be processed or being processed (e.g., cooked, chopped, sliced etc.). The determined measure may be used to control the operation of the food processing device, e.g., to achieve a desired food processing outcome (e.g., a desired cooking outcome or slicing outcome). In particular, the determined measure could be used as feedback to control the parameter during food processing. As another example, the determined measure may be displayed at a display. As yet another example, the determined measure may be provided to a further processing device for further processing, e.g., to determine future cooking/processing parameters or the like.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

The mere fact that certain measures are recited in mutually different dependent claims does not indicate that a combination of these measures cannot be used to advantage.

If the term "adapted to" is used in the claims or description, it is noted the term "adapted to" is intended to be equivalent to the term "configured to".

Any reference signs in the claims should not be construed as limiting the scope.

## Claims

1. An apparatus (100, 200) for sensing the value of a physical parameter at a sensing location, the apparatus comprising:
a transmitter element (110, 210) configured to generate and transmit a pulsed electromagnetic wave (300);
a sensing element (120, 220), galvanically isolated from the transmitter element and configured to be positioned at or adjacent to the sensing location, wherein:
the sensing element comprises a capacitive element (C) and a resistive element (R) connected in a loop such that, responsive to the sensing element receiving the pulsed electromagnetic wave, the sensing element generates an oscillating electromagnetic wave (400) that changes responsive to an impedance of the capacitive element and/or resistive element; and
the capacitive and/or resistive element is configured such that an impedance of the capacitive and/or resistive element changes responsive to changes in the physical parameter in the vicinity of the sensing element; and
a detection element (130, 210), galvanically isolated from the sensing element, and configured to detect the oscillating electromagnetic wave and process the detected oscillating electromagnetic wave to estimate a value of the physical parameter at the sensing location.

2. The apparatus (100, 200) of claim 1, wherein the detection element is configured to calculate the time damping constant of the detected oscillating electromagnetic wave and process the calculated time damping constant to estimate the value of the parameter at the sensing location.

3. The apparatus (100, 200) of claim 1 or 2, wherein the detection element (130, 210) is configured to integrate a reference signal between two time points defined by an upper or lower envelope of the detected oscillating electromagnetic wave to produce an integrated value for the oscillating electromagnetic wave and process the integrated value to estimate the value of the parameter at the sensing location.

4. The apparatus (100, 200) of any of claims 1 to 3, wherein:
the transmitter element (110, 210) comprises a transmitter coil (111, 211) for transmitting the pulsed electromagnetic wave; and
the detection element (130, 210) comprises a detector coil (131, 211) for detecting the oscillating electromagnetic wave.

5. The apparatus (200) of claim 4, wherein the transmitter coil and detection coil are together formed from a single coil (211) for transmitting the pulsed electromagnetic wave and detecting the oscillating electromagnetic wave.

6. The apparatus (100, 200) of any of claims 1 to 5, wherein the resistive element (R) is a thermistor, such that the physical parameter is a temperature.

7. The apparatus (100, 200) of any of claims 1 to 6, wherein the pulsed electromagnetic wave (300) is a square pulsed electromagnetic wave.

8. The apparatus (100, 200) of any of claims 1 to 7, wherein the sensing element (120, 220) comprises a sensing coil (221) connected in the loop with the capacitive element and the resistive element for receiving the pulsed electromagnetic wave and outputting the oscillating electromagnetic wave.

9. A cooking device (600) comprising the apparatus of any of claims 1 to 8, wherein the sensing element (120, 220) is positioned such that the sensing location is in or in the vicinity of a food item (690) in the cooking device.

10. The cooking device (600) of claim 9, comprising a pan (620) for housing the food item,
wherein the transmitter element (111, 113, 121) and the detection element are positioned externally to the pan and the sensing element (120, 220) is configured to be housed within the pan.

11. The cooking device (600) of claim 10, wherein the pan (620) is non-metallic.

12. The cooking device (600) of claim 10 or 11, comprising a housing (650) and a moveable support (630) for supporting the pan, wherein the housing and moveable support are configured such that the moveable support, when supporting the pan, is able to move between:
a first position in which any food item housed in the pan, is enclosed by the housing, the pan and the moveable support; and
a second position in which the pan is exposed for accessing any food item housed in the pan.

13. The cooking device (600) of claim 12, when dependent upon claim 4, wherein the transmitter coil (111, 211) and the detector coil (131, 211) are mounted to the moveable support.

14. The cooking device (600) of claim 12, when dependent upon claim 4, wherein the transmitter coil (111, 211) and the detector coil (131, 211) are integrated into or positioned on the housing.

15. The cooking device (600) of any of claims 10 to 14, wherein:
the pan comprises a planar structure configured, in use, to support the food item in the cooking device, the planar structure lying in a first plane; and
the transmitter coil and detector coil are positioned to lie in a second plane that is perpendicular to the first plane.
